# EUROPEAN PATENT APPLICATION

(11) **EP 3 967 252 A1**
(43) Date of publication of application: **16.03.2022**
(21) Application number: 21195021.7
(22) Date of filing: 06.09.2021
(51) Int. Cl.: A61B 18/14, A61B 17/00, A61B 17/16, A61B 18/00

(54) **MULTIFUNCTIONAL SURGICAL TOOL AND SYSTEM**

(30) Priority: 15.09.2020 US 202063078724 P; 20.08.2021 US 202117407637
(71) Applicant: Mazor Robotics Ltd., 3088900 Caesarea (IL)
(72) Inventor: ZUCKER, Ido, 3079567 Caesarea (IL); MANLEY, Prakash, 3079567 Caesarea (IL); LAOUAR, Yahia, 3079567 Caesarea (IL); CHENG, Xiaoming, 3079567 Caesarea (IL); MIRI, Mohammad, 3079567 Caesarea (IL); PALM, Matthew, 3079567 Caesarea (IL)
(74) Representative: Viering, Jentschura & Partner mbB Patent- und Rechtsanwälte

(57) **Abstract**

A surgical tool may include a mechanical tool for selectively cutting or removing anatomical tissue, and an electrosurgery device selectively operable to apply an electric current to anatomical tissue. The mechanical tool may include a tool bit and a driver operatively connected to the tool bit, and the electrosurgery device may include a first electrode and a second electrode different than the first electrode.

## Description

### CROSS REFERENCE TO RELATED APPLICATIONS

This application claims the benefit of U.S. Provisional Application No. 63,078,724, filed on September 15, 2020, and entitled "Multifunctional Surgical Tool and System", which application is incorporated herein by reference in its entirety.

### FIELD

The present technology generally relates to surgical tools, and more particularly relates to surgical tools for providing both mechanical and electrosurgical capabilities.

### BACKGROUND

Various surgical tools are often required to successfully complete surgical procedures. Certain types of tools may be required for different procedures. Mechanical surgical tools include drills, burrs, saws, shavers, scalpels, reamers, and taps. Electrosurgical tools utilize applied electrical current for cauterization, ablation, fulguration, and desiccation. Electrosurgical tools may include monopolar or bipolar electrode models.

### SUMMARY

Example aspects of the present disclosure include:

A surgical tool according to at least one embodiment of the present disclosure comprises a mechanical tool for selectively cutting or removing anatomical tissue, and an electrosurgery device selectively operable to apply an electric current to anatomical tissue. The mechanical tool comprises a tool bit; and a driver operatively connected to the tool bit. The electrosurgery device comprises a first electrode; and a second electrode different than the first electrode.

Any of the aspects herein, wherein at least one of the first electrode and the second electrode is retractable from a first position used when the electrosurgery device is operated to a second position used when the mechanical tool is operated.

Any of the aspects herein, wherein the first electrode is the tool bit.

Any of the aspects herein, wherein the tool bit is insulated from the first electrode and the second electrode.

Any of the aspects herein, wherein activation of the driver deactivates the electrosurgery device.

Any of the aspects herein, wherein the driver and the electrosurgery device are operable simultaneously.

Any of the aspects herein, wherein the tool bit comprises a drill bit, a burr, a saw, an ultrasonic scalpel, or a shaver.

Any of the aspects herein, further comprising a fluid conduit for discharging fluid proximate a distal end of the electrosurgery device.

Any of the aspects herein, wherein extension of the tool bit causes at least one of the first electrode and the second electrode to move from an operating position to a non-operating position.

Any of the aspects herein, wherein the at least one of the first electrode and the second electrode is biased toward the operating position.

A dual-mode surgical system comprising: a housing having a proximal end and a distal end; a mechanical tool bit supported at least partially within the housing; an electrosurgery device supported by the housing, the electrosurgery device comprising at least one electrical (e.g., RF) conductor; and a fluid conduit for channeling fluid to proximate the distal end.

Any of the aspects herein, further comprising a valve for selectively releasing fluid from the fluid conduit.

Any of the aspects herein, wherein the mechanical tool bit comprises an electrode of the electrosurgery device.

Any of the aspects herein, wherein the mechanical tool bit is movable between a retracted position and an extended position.

Any of the aspects herein, wherein the at least one electrical (e.g., RF) conductor comprises first and second elements hingedly connected to the housing, each of the first and second elements moveable between an open position, in which the first element does not contact the second element, and a closed position, in which the first element is in contact with the second element.

Any of the aspects herein, wherein the first and second elements are biased toward the closed position.

A surgical tool according to at least one embodiment of the present disclosure comprises a housing; a first tool bit operably connected to a motor and supported by the housing, the first tool bit in electrical contact with a first electrical lead; a second tool bit supported by the housing and insulated from the first tool bit within the housing, the second tool bit in electrical contact with a second electrical lead different than the first electrical lead; and a user interface operable to select a non-powered mode in which the second tool bit may be used to modify anatomical tissue, a first powered mode in which the first tool bit may be used to modify anatomical tissue, or a second powered mode in which the first tool bit and the second tool bit are used to apply electrical current to anatomical tissue.

Any of the aspects herein, wherein one of the first tool bit and the second tool bit comprises a fluid conduit for discharging fluid proximate a working end thereof.

Any of the aspects herein, wherein the first tool bit is a spine shaver and the second tool bit is a curette.

Any of the aspects herein, wherein the spine shaver comprises a fluid conduit operable to discharge fluid in the second powered mode.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/embodiments in combination with any one or more other aspects/features/embodiments.

Use of any one or more of the aspects or features as disclosed herein.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2 illustrates a cross-section of a surgical tool according to at least one embodiment of the present disclosure;
Fig. 3A illustrates a cross-section of a surgical tool in an extended position according to at least one embodiment of the present disclosure;
Fig. 3B illustrates a cross-section of the surgical tool of Fig. 3A in a retracted position according to at least one embodiment of the present disclosure;
Fig. 4 illustrates a distal end portion of a surgical tool according to at least one embodiment of the present disclosure; and
Fig. 5A illustrates a side view of a surgical tool according to at least one embodiment of the present disclosure; and
Fig. 5B illustrates a side view of a surgical tool according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

In orthopedic surgery, a surgeon may use power tools to cut, drill, or burr down bone. These power tools may include rotating and/or oscillating tools. The rotating and/or oscillating tools may rotate and/or oscillate at various frequencies, including ultrasonic frequency ranges. During surgery, the surgeon may first expose a bone by removing any soft tissue covering the bone. However, the interaction between the power tools and the soft tissue may result in degradation of the efficiency of the power tool, clogging of the power tool, or the pulling of soft tissue, which may be harmful to the patient. Moreover, the power tool may be designed to be tissue selective and, as a result, may not penetrate through soft tissue.

A surgical tool according to embodiments of the present disclosure may contain a mechanical tool (e.g., a drill, burr, saw, oscillating saw, tap, ultrasonic tool, etc.) combined with an electrosurgical component configured to perform electrosurgery. The electrosurgical component may be or comprise a bipolar or a monopolar configuration. The surgical tool may enable the activation of the electrosurgical component to assist with, among other things, the ablation of soft tissue covering the path of the mechanical tool. As such, the electrosurgical component may clear a path for the mechanical tool. Additionally or alternatively, the electrosurgical component may be utilized to stop patient bleeding.

The surgical tool may use a mechanical tool tip (e.g., a drill, burr, saw, oscillating saw, tap, ultrasonic tool, etc.) as the electrosurgical component by electrically isolating the mechanical tool tip. In other words, the mechanical tool tip can be isolated from any conductive surface and configured to perform electrosurgical tasks. Additionally or alternatively, the mechanical tool tip may have additional elements that may protrude from the surgical tool to provide electrosurgical functionality.

Inter-vertebral discs are prepared prior to vertebral fusion by removing the fibrous/gelatinous nucleus as well as the inner annulus material contained therein. During disc preparation, quick removal of the material may be preferred, while the number of passes over the spinal cord and the nerve ganglia may be minimized. Additionally, disc preparation may sufficiently prepare the cartilage endplates and expose the bleeding bone without damaging the endplates or exposing the trabecular bone. Furthermore, in some embodiments, the annulus is not perforated during the disc preparation. Though in other embodiments, the annulus may be initially perforated for access to prepare the disc.

A surgical tool according to embodiments of the present disclosure may implement powered spine shaver technology with radio frequency (RF) capabilities. The surgical tool may administer an RF pre-treatment step before applying the spine shaver during the disc preparation. Additionally or alternatively, the surgical tool may contain or comprise a bipolar spine shaver device that allows deployment of RF independently of the spine shaver, allowing the surgical tool to operate as a cold scraper. The surgical tool may comprise a curette, which may act as one of the RF electrodes, while the cutting blades of the spine shaver may act as the other electrode to perform bipolar electrosurgery. The surgical tool may additionally contain a fluid reservoir capable of flushing saline out of the spine shaver. The flushed saline may facilitate the movement and operation of the surgical tool, as well as facilitate material removal.

Such a surgical tool may provide low dose RF power through a spine shaver handset modified to treat the spine shaver as a return electrode. The curette may function as the primary RF electrode, which can either be used for RF delivery or for material removal as desired by the operator of the surgical tool. By applying RF pre-treatment, the collagen contained within the disc may be transformed into a form that can be more easily cut and removed by the spine shaver device.

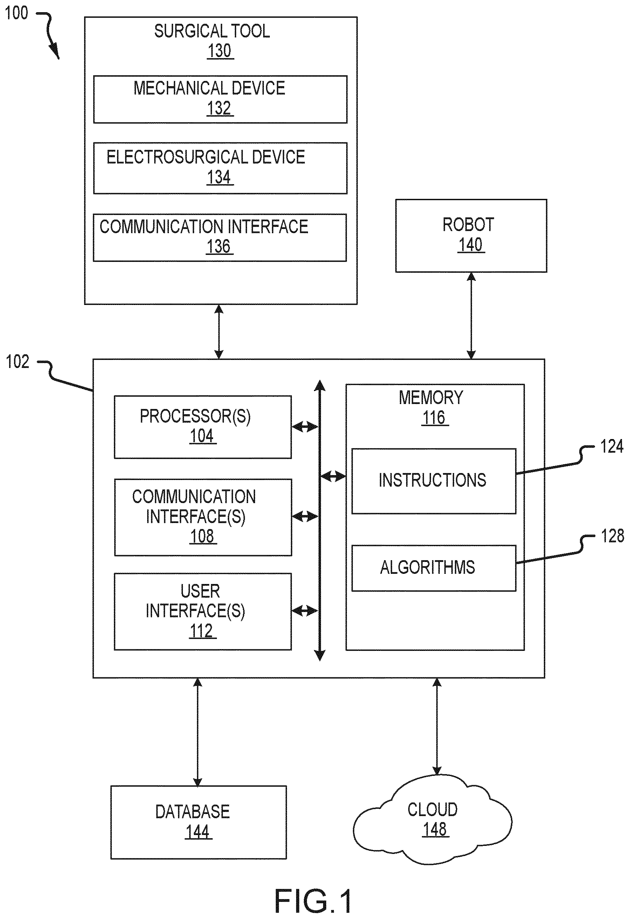

Turning now to Fig. 1, a block diagram of a system 100 according to at least one embodiment of the present disclosure is shown. The system 100 may be used, for example, to carry out a procedure utilizing a multifunction surgical tool as described herein, or to gather information relevant to such a procedure; to improve patient outcomes in connection with a surgical procedure or task; or for any other useful purpose. The system 100 comprises a computing device 102, a surgical tool 130, a robot 140, a database 144, and a cloud 148. Notwithstanding the foregoing, systems according to other embodiments of the present disclosure may omit any one or more of the robot 140, database 144, and/or the cloud 148. Additionally, systems according to other embodiments of the present disclosure may arrange one or more components of the system 100 differently (e.g., the surgical tool 130 may comprise one or more of the components of the computing device 102, and/or vice versa).

The computing device 102 comprises at least one processor 104, at least one communication interface 108, at least one user interface 112, and at least one memory 116. A computing device according to other embodiments of the present disclosure may omit one or both of the communication interface(s) 108 and/or the user interface(s) 112.

The at least one processor 104 of the computing device 102 may be any processor identified or described herein or any similar processor. The at least one processor 104 may be configured to execute instructions 124 stored in the at least one memory 116, which instructions 124 may cause the at least one processor 104 to carry out one or more computing steps utilizing or based on data received, for example, from the surgical tool 130, the robot 140, the database 144, and/or the cloud 148. The instructions 124 may also cause the at least one processor 104 to utilize one or more algorithms 128 stored in the memory 116. In some embodiments, the at least one processor 104 may be used to control the surgical tool 130 and/or the robot 140 during a surgical procedure, including during a procedure being carried out autonomously or semi-autonomously by the robot 140 using the surgical tool 130.

The computing device 102 may also comprise at least one communication interface 108. The at least one communication interface 108 may be used for receiving sensor data (e.g., from the surgical tool 130), a surgical plan or other planning data, or other information from an external source (such as the surgical tool 130, the robot 140, the database 144, the cloud 148, and/or a portable storage medium (e.g., a USB drive, a DVD, a CD)), and/or for transmitting instructions, images, or other information from the at least one processor 104 and/or the computing device 102 more generally to an external system or device (e.g., another computing device 102, the surgical tool 130, the robot 140, the database 144, the cloud 148, and/or a portable storage medium (e.g., a USB drive, a DVD, a CD)). The at least one communication interface 108 may comprise one or more wired interfaces (e.g., a USB port, an ethernet port, a Firewire port) and/or one or more wireless interfaces (configured, for example, to transmit information via one or more wireless communication protocols such as 802.1 1a/b/g/n, Bluetooth, Bluetooth low energy, NFC, ZigBee, and so forth). In some embodiments, the at least one communication interface 108 may be useful for enabling the device 102 to communicate with one or more other processors 104 or computing devices 102, whether to reduce the time needed to accomplish a computing-intensive task or for any other reason.

The at least one user interface 112 may be or comprise a keyboard, mouse, trackball, monitor, television, touchscreen, button, joystick, switch, lever, and/or any other device for receiving information from a user and/or for providing information to a user of the computing device 102. The at least one user interface 112 may be used, for example, to receive a user selection or other user input; to receive a user selection or other user input regarding one or more configurable settings of the computing device 102, the surgical tool 130, the robot 140, and/or of another component of the system 100; to receive a user selection or other user input regarding how and/or where to store and/or transfer data received, modified, and/or generated by the computing device 102; and/or to display information (e.g., text, images) and/or play a sound to a user based on data received, modified, and/or generated by the computing device 102.

Although the at least one user interface 112 is shown as part of the computing device 102, in some embodiments, the computing device 102 may utilize a user interface 112 that is housed separately from one or more remaining components of the computing device 102. In some embodiments, the user interface 112 may be located proximate one or more other components of the computing device 102, while in other embodiments, the user interface 112 may be located remotely from one or more other components of the computer device 102.

The at least one memory 116 may be or comprise RAM, DRAM, SDRAM, other solid-state memory, any memory described herein, or any other tangible non-transitory memory for storing computer-readable data and/or instructions. The at least one memory 116 may store, for example, instructions 124 and/or algorithms 128. In some embodiments, the memory 116 may also store one or more preoperative and/or other surgical plans; one or more images of one or more patients, including in particular of an anatomical feature of the one or more patients on which one or more surgical procedures is/are to be performed; data received from the surgical tool 130 (including any component thereof) or elsewhere; and/or other information useful in connection with the present disclosure.

The instructions 124, as described above, may be or comprise any instructions for execution by the at least one processor 104 that cause the at least one processor to carry out one or more steps of any of the methods described herein. The instructions 124 may be or comprise instructions for carrying out a procedure. The instructions 124 may additionally or alternatively enable the at least one processor 104, and/or the computing device 102 more generally, to increase the likelihood of a positive procedural outcome during any surgical procedure in which information obtained from a surgical tool as described herein may be relevant.

The algorithms 128 may be or comprise any algorithms useful for converting sensor data received from the surgical tool 130 into meaningful information (e.g., a calculated force value, pressure value, distance measurement). The algorithms 128 may further be or comprise any algorithms useful for generating one or more recommendations to a surgeon or other user of the system 100 based on information received from a surgical tool 130, and/or for modifying a preoperative or other surgical plan based on such information and/or an evaluation of such information. The algorithms 128 may further be or comprise algorithms useful for controlling the surgical tool 130 and/or the robot 140. In some embodiments, the algorithms 128 may be or include machine learning algorithms.

The surgical tool 130 comprises a mechanical device 132, an electrosurgical device 134, and a communication interface 136. The surgical tool 130 is adapted to, among other things, perform a surgical operation during a surgical procedure, and can communicate with the computing device 102, the robot 140, the database 144, and/or the cloud 148. In some embodiments, the surgical tool comprises additional mechanical devices and/or electrosurgical tools, such that the surgical tool is capable of performing various surgical tasks. For example, in some embodiments, the surgical tool 130 may comprise two mechanical devices (e.g., a drill and a burr) and an electrosurgical device (e.g., an electrocautery device). In this instance, the surgical tool 130 may be capable of using the drill and the burr, while also capable of activating the electrocautery device. Also in some embodiments, the mechanical device 132 may comprise a motor and a shaft to which one of a plurality of tools may be operatively and removably secured, such as a drill bit, a burr, a screwdriver, or any other rotating tool bit. The surgical tool 130 may be operated manually or automatically. The surgical tool 130 may be utilized by a surgeon or may be controlled by the computing device 102 while a surgical procedure is carried out by the robot 140. In some embodiments, the system 100 may comprise more than one surgical tool 130.

The mechanical device 132 of the surgical tool 130 is configured to perform a mechanical surgical task. For instance, the mechanical device 132 may be a drill designed to drill through anatomical tissue. In some embodiments, the mechanical device 132 may perform different mechanical surgical tasks. For example, the mechanical device 132 may be designed, shaped, or otherwise configured to perform drilling, burring, shaving, sawing, cutting, reaming, penetrating, and/or tapping tasks on anatomical tissue.

The electrosurgical device 134 of the surgical tool 130 is configured to perform electrosurgery. Electrosurgery may be implemented by the electrosurgical device 134, for example, to supplement the conventional approach of cutting though anatomical tissue using mechanical tools. During surgery, the surgeon may make use of electrosurgery for various purposes, such as to divide or desiccate anatomical tissue, as well as to coagulate bleeding (e.g., through fulguration of the bleeding anatomical tissue). Electrosurgery may be implemented through two different electrode configurations: monopolar and bipolar. In monopolar electrosurgery, an active electrode is placed at a surgical site. The return electrode is placed somewhere else on the body of a patient. Once the circuit is powered, current flows through the active electrode to the return electrode, with the current passing through the body of the patient. The current passing through the body of the patient creates the desired electrical effect at the surgical site, based on the current amount, density and the waveform of the current. Alternatively, bipolar electrosurgery may be performed. In bipolar electrosurgery, the return electrode is a component of the surgical tool, rather than a separate component positioned elsewhere on the body of the patient. Since the surgical tool contains both electrodes, no return electrode located on the body of the patient is required; instead, the current passes through any tissue located between and in proximity of the active electrode and the return electrode of the surgical tool.

By modulating the waveform generated by the power source, the target site experiences different effects. For instance, by using a waveform such as a continuous sine waveform, the surgeon is able to vaporize or cut tissue at the target site. By using an intermittent waveform, the electrosurgery produces more heat momentarily, allowing for coagulation of the target site. By increasing the intermittence of the waveform, the surgeon is able to create a heat spectrum between the electrodes, which may be used to perform various levels of coagulation and/or tissue vaporization.

The communication interface 136 may be the same as or similar to the communication interface 108. For example, the communication interface 136 may be utilized for receiving operating instructions and/or control signals from an external source (such as the computing device 102, the robot 140), and/or for transmitting data (e.g., corresponding to one or more measurements made by the surgical tool 130) or other information to an external system or device (e.g., the computing device 102, the robot 140, the database 144, the cloud 148, and/or a portable storage medium (e.g., a USB drive, a DVD, a CD)). The communication interface 136 may comprise one or more wired interfaces (e.g., a USB port, an ethernet port, a Firewire port) and/or one or more wireless interfaces (configured, for example, to transmit information via one or more wireless communication protocols such as 802.1 1a/b/g/n, Bluetooth, Bluetooth low energy, NFC, ZigBee, and so forth). In some embodiments, the communication interface 136 may be useful for enabling the device 102 to communicate with one or more other processors 104 or computing devices 102, whether to reduce the time needed to accomplish a computing-intensive task or for any other reason.

The robot 140 may be any surgical robot or surgical robotic system. The robot 140 may be or comprise, for example, the Mazor X^{™} Stealth Edition robotic guidance system. The robot 140 may comprise a base that supports a robotic arm configured to hold the surgical tool 130. The robot 140 may comprise one or more robotic arms, each of which may be configured to hold the surgical tool 130. The robot 140 may, in some embodiments, assist with a surgical procedure (e.g., by holding a tool in a desired trajectory or pose and/or supporting the weight of a tool while a surgeon or other user operates the tool, or otherwise) and/or automatically carry out a surgical procedure. The robot 140 may comprise one or more sensors useful for gathering information about where the robot or any portion thereof is positioned relative to a patient or, more specifically, relative to a patient's spine. The robot 140 may further comprise one or more sensors useful for assisting the robot 140 to determine whether it has good purchase and/or is positioned correctly.

The database 144 may store any information that is shown in Fig. 1 as being stored in the memory 116, including instructions such as the instructions 124 and/or algorithms such as the algorithms 128. In some embodiments, the database 144 stores one or more preoperative or other surgical plans. The database 144 may additionally or alternatively store, for example, information about or corresponding to one or more characteristics of the surgical tool 130 and/or other information regarding available tools and/or equipment for use in connection with a surgical procedure. The database 144 may be configured to provide any such information to the computing device 102, the surgical tool 130, the robot 140, or to any other device of the system 100 or external to the system 100, whether directly or via the cloud 148. In some embodiments, the database 144 may be or comprise part of a hospital image storage system, such as a picture archiving and communication system (PACS), a health information system (HIS), and/or another system for collecting, storing, managing, and/or transmitting electronic medical records including image data. Also in some embodiments, the memory 116 may store any of the information described above.

The cloud 148 may be or represent the Internet or any other wide area network. The computing device 102 may be connected to the cloud 148 via the communication interface 108, using a wired connection, a wireless connection, or both. In some embodiments, the computing device 102 may communicate with the database 144 and/or an external device (e.g., a computing device) via the cloud 148.

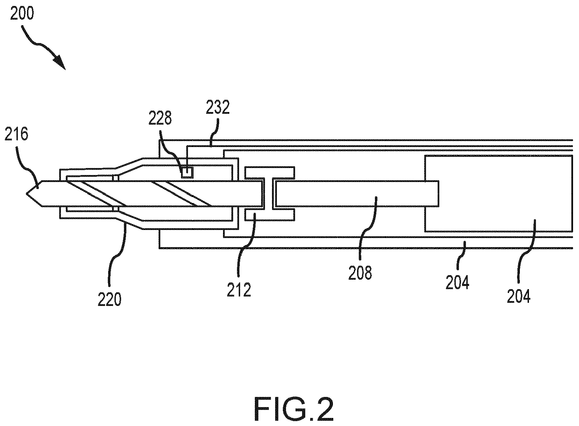

Referring now to Fig. 2, a surgical tool 200 in accordance with embodiments of the present disclosure comprises a motor 204, a rotating shaft 208, a rotating insulator 212, a tool bit 216, an insulative support 220, a housing 224, an RF lead 228, and a wire 232. Notwithstanding the layout of the surgical tool 200 as illustrated in Fig. 2, surgical tools according to other embodiments of the present disclosure may comprise more or fewer components than the surgical tool 200. In some embodiments, for example, the RF lead 228 may have a complementary electrode within the surgical tool 200 such that the surgical tool 200 is capable of performing bipolar electrosurgery.

The motor 204 receives power from a power source (not shown) and converts the power into mechanical energy to operate the tool bit 216 of the surgical tool 200. The motor 204 is disposed within a proximal end of the housing 224 of the surgical tool 200. The motor 204 is operatively attached to a proximal end of the rotating shaft 208 and causes rotary motion of the rotating shaft 208, the rotating insulator 212, and the tool bit 216. As used above and herein, the term "proximal end" refers to the end of a tool or part, or a location, proximate an operator of a surgical tool, and the term "distal end" or "working end" refers to the end of the tool or part, or a location, proximate a patient. In some embodiments, the motor 204 may vary in type and design to best match the requirements of the surgery being performed by the surgical tool 200. Some non-limiting examples of motor types that may be used include a DC brushed motor, an AC or DC brushless motor, a direct drive motor, a servo motor, a stepper motor, a turbine motor, a pneumatic motor, or the like. In some embodiments, the motor 204 may provide linear motion instead of or in addition to rotating motion in the rotating shaft 208, such as when the tool bit 216 of the surgical tool 200 requires linear motion perform an operation. In this case, the motor 204 may be a linear motor designed to provide translational motion in the rotating shaft 208, or the motor 204 may be designed to create both translational and rotational motion in the rotating shaft 208, the rotating insulation 212, and/or the tool bit 216. In some embodiments, one or more gears or other mechanical devices (e.g., a rack and pinion) may be used to convert rotational motion (e.g., of a shaft 208 attached to a motor 204) into translational motion, and/or vice versa.

The rotating insulator 212 of the surgical tool 200 is located within the housing 224 and operatively attached to a distal end of the rotating shaft 208. The rotating insulator 212 is configured to receive a portion of a proximal end of the tool bit 216 and to transfer torque/rotational energy from the rotating shaft 208 to the tool bit 216. The rotating insulator 212 also provides a physical and electrical separation between the rotating shaft 208 and the tool bit 216, which assists in preventing electrical current from flowing improperly through the surgical tool 200. For example, in the event that the tool bit 216 receives electrical current (such as during the performance of electrosurgery), the rotating insulator 212 prevents the electrical current from flowing through the rotating shaft 208 and into the motor 204. The prevention of electrical current flow back in a proximal direction toward the motor 204 lowers the probability of damage to the motor 204 and reduces the chance of short circuits within the surgical tool 200.

The rotating insulator 212 is constructed from an insulative material designed to prevent electrical current from passing therethrough. In some embodiments, the rotating insulator 212 may uniformly comprise a single insulative material. For instance, the rotating insulator 212 may be made entirely of a plastic. Alternatively, in some embodiments the rotating insulator 212 may contain a mixture or combination of different types of insulative material. For example, the rotating insulator 212 may comprise a combination of glass or ceramic and plastic. Additionally or alternatively, the rotating insulator 212 may contain an insulative coating on the surface thereof. The insulative coating may comprise the same material as or a different material than that of the insulative material. The rotating insulator 212 may comprise, by way of example, insulative material and/or an insulative coating made of glass, plastic, porcelain, synthetic resins, paper, Teflon, rubber (natural and/or synthetic), PVC, and/or combinations thereof.

The rotating insulator 212 may be rotationally symmetric about a center axis thereof. In some embodiments, however, to facilitate the transfer of torque or rotational energy from the shaft 208 to the tool bit 216, the rotating insulator 212 may comprise one or more flats on an interface with the shaft 208 (e.g., on the proximal side of the rotating insulator 212), and/or on an interface with the tool bit 216 (e.g., on the distal side of the rotating insulator 212). For example, in some embodiments, the rotating insulator 212 may comprise a hex socket to receive a hex-shaped end of either or both of the shaft 208 and the drill bit 216. Other shapes may alternatively be used to prevent the rotating insulator 212 from slipping relative to the shaft 208 and/or the tool bit 216.

The tool bit 216 of the surgical tool 200 is at least partially located in the housing 224 of the surgical tool 200 and comprises a proximal end connected to the rotating insulator 212 and a distal end extending out of the housing 224. The tool bit 216 is configured to rotate in response to rotational movement from the rotating insulator 212. The distal end of the tool bit 216 provides a form of mechanical utility for the performer of the surgical procedure. For example, in some embodiments, the distal end of the tool bit 216 may be a drill bit configured to facilitate drilling through anatomical tissue.

The distal end of the tool bit 216 is not limited to the drill bit mentioned above, and other types, shapes, and/or forms of the distal end of the tool bit 216 may be utilized. The distal end of the tool bit 216 may be designed, shaped, or otherwise formed to drill, burr, shave, saw, cut, ream, penetrate, and/or tap through anatomical tissue. In some embodiments, the dimensions of the tool bit 216 may vary, for example, depending on the type of surgical procedure, surgeon preference, and/or combinations thereof. Accordingly, the surgical tool 200 may be designed to accommodate and accept various tool bit sizes, shapes, and forms. For example, the tool bit 216 may in some embodiments extend from a proximal end of the housing by less than one inch, or between one inch and two inches, or between two inches and five inches, or between five inches and ten inches, or by more than ten inches. In some embodiments, surgical tool 200 may be configured to accept more than one tool bit 216 (albeit not at the same time), where each tool bit has a different distal end shape, but a similar proximal end shape. For instance, a first tool bit may be a saw, while a second tool bit may be a drill; the first and second tool bits may have different distal end shapes (i.e., corresponding to a saw and a drill, respectively), but may have similar proximal end shapes (e.g., shapes that enable the proximal end to be received in the rotating insulator 212) such that the surgical tool 200 is configured to accept either the first or second tool bit without requiring any significant reconfiguration. In some embodiments, the distal end of the tool bit 216 may be designed to selectively target different forms of anatomical tissue. For example, the distal end of the tool bit 216 may be a saw designed to cut through bone.

As shown in Fig. 2, the surgical tool 200 has the insulative support 220 circumferentially disposed around the tool bit 216. The insulative support 220 both supports the tool bit 216 within the housing 224 (thus helping to ensure that the tool bit 216 rotates around its center axis and thus facilitating proper use thereof), and prevents the tool bit 216 from directly contacting the housing 224 (thus ensuring that the tool bit 216 is electrically isolated from the housing 224, so as to protect, for example, the user of the surgical tool 200 from possible electric shock). The insulative support 220 may comprise the same material as or a similar material to that of the rotating insulator 212. The insulative support 220 may be rigid or flexible, and may provide structural support to and assist in the alignment of the tool bit 216 within the surgical tool 200.

The RF lead 228 of the surgical tool 200 contacts the tool bit 216 and is located radially within the circumferentially disposed insulative support 220. The RF lead 228 provides an electrical contact to the tool bit 216, allowing for current flow to the tool bit 216. The RF lead 228 is connected to the wire 232 extending through the housing 224 and running to the proximal end of the surgical tool 200. The wire 232 is electrically insulated from the housing 224 such that the housing 224 does not conduct the electricity that flows through the wire 232. The wire 232 permits current flow from a power source to the RF lead 228, and as such allows the surgical tool 200 to be used to perform monopolar electrosurgery. In some embodiments, the RF lead 228 may be retractable from the tool bit 216. In such embodiments, when the tool bit 216 is being powered by the motor 204, the RF lead 228 may be in a retracted position such that the RF lead 228 does not contact the tool bit 216 when the tool bit 216 is in motion. Upon the surgical tool 200 switching to use of monopolar electrosurgery, the RF lead 228 may move from the retracted position to an extended position. In the extended position, the RF lead 228 may contact the tool bit 216, allowing the surgical tool 200 to be used to perform monopolar electrosurgery.

In other embodiments, the RF lead 228 may remain in contact with the tool bit 216, regardless of whether the surgical tool 200 is being used for monopolar electrosurgery or not. In such embodiments, a physical switch may be used to prevent inadvertent flow of electricity to the RF lead 228 and the drill bit 216 when the operator of the surgical tool 200 is not using the surgical tool 200 for electrosurgery. Also in such embodiments, the RF lead 228 may be biased toward the drill bit 216, so that wear resulting from the contact of the drill bit 216 and the RF lead 228 does not break the connection between the drill bit 216 and the RF lead 228. For example, a spring may be placed between the insulative support 220 and the RF lead 228, so as to push the RF lead 228 toward the drill bit 216. The RF lead 228 may be or comprise metal or any other conductive material, and may be formed as a brush, a pad, or any other shape suitable for facilitating contact between the drill bit 216 and the RF lead 228.

To perform the monopolar electrosurgery, the operator of the surgical tool 200 turns on the power source connected to the wire 232. In some embodiments, turning on the power source simply comprises establishing an electrical current flow path from the power source to the wire 232 (e.g., using a manual or electronic switch). The power source is capable of supplying the necessary current for performance of electrosurgery to the RF lead 228 and thus to the tool bit 216. In some embodiments, the power source may be located external to the surgical tool 200, with the surgical tool 200 wired to the power source. In some embodiments, the power source may be located within the surgical tool 200, and the surgical tool 200 may be outfitted with a button, switch, lever, or the like to permit the surgeon or other operator to activate the power source. In some embodiments, the activation of the power source may deactivate the motor 204 such that the tool bit 216 no longer translates and/or rotates. In some embodiments, the surgical tool 200 may prevent the activation of electrosurgery until the rotation speed of the tool bit 216 is below a threshold level. The threshold level may be determined by the computing device 102, which may prevent the power source from sending current through the wire 232 until the rotation speed of the tool bit 216 is below the threshold level. In other embodiments, the surgical tool 200 may be operable to simultaneously drive the tool bit 216 using the motor 204 and to provide electrical current to the tool bit 216 via the RF lead 228.

When the power source is activated, electrical current can flow through the wire 232 to the RF lead 228 and into the tool bit 216. To complete the circuit, a return electrode can be positioned on, around, near, or inside the body of a patient such that a surgical site that is the desired target of the monopolar electrosurgery is positioned along a current flow path from the tool bit 216 to the return electrode. The return electrode completes the circuit and permits current flow from the tool bit 216 through the body of the patient, including the surgical site, and out through the return electrode. Once the monopolar electrosurgical operation is complete, the power source can be turned off (e.g., by a surgeon or operator of the surgical tool 200), and the motor 204 can be reactivated to operate the mechanical utility of the surgical tool 200 (i.e., the operation of the tool bit 216). In some embodiments, the motor 204 is powered by the same power source as the RF lead 228, and a multi-position switch is used to control whether the power source provides electricity to the motor 204 only, to the RF lead 228 only, to both the motor 224 and the RF lead 228, or to neither the motor 224 nor the RF lead 228.

As noted above, the surgical tool 200 may contain a switch, which permits a surgeon or other operator to switch the surgical tool 200 between a mechanical function and an electrosurgical function. During the mechanical function, the motor 204 induces rotational movement in the tool bit 216, permitting the tool bit 216 to selectively cut or remove anatomical tissue. When electrosurgery is required or would be beneficial during the surgery, the surgeon may switch the surgical tool 200 to the electrosurgical function. As a result, the motor 204 may be switched off and the tool bit 216 may no longer mechanically operate. The surgical tool 200 may then be used by the surgeon to perform monopolar electrosurgery by positioning the surgical tool 200 such that the surgical site is between the tool bit 216 and the return electrode. The resulting current though the wire 232 to the RF lead 228 may then pass through the tool bit 216 and through the patient to the return electrode, creating the desired electrosurgical effect.

In some embodiments, the surgical tool 200 may permit both the mechanical function and the electrosurgery function to operate simultaneously. In other words, the mechanical operation performed by the tool bit 216 may function independently of the electrosurgery. To perform electrosurgery, the power source may be turned on, and current may still flow through the surgical tool 200 and the patient as described above while the tool bit 216 is still receiving translational and/or rotational energy from the motor 204.

While the surgical tool 200 of Fig. 2 may be outfitted to perform monopolar electrosurgery, the surgical tool 200, as well as other embodiments of the present disclosure, are in no way limited to performance of monopolar electrosurgery, and the surgical tool 200 may be provided with an additional electrode, insulated from the RF lead 228 and the tool bit 216, to permit the surgical tool 200 to alternatively perform bipolar electrosurgery.

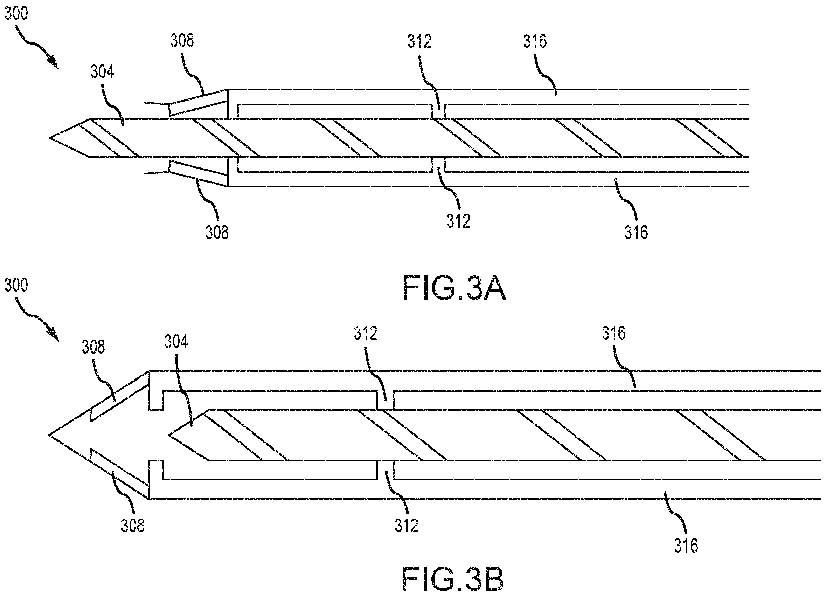

Figs. 3A and 3B illustrate cross sections of a working end of a surgical tool 300 in an extended and a retracted position, respectively, in accordance with embodiments of the present disclosure. The surgical tool 300 may be the same as or similar to the surgical tool 200. In some embodiments, the surgical tool 300 may comprise a motor, a shaft, and a rotating insulator to cause movement of a tool bit 304. The motor may be the same as or similar to the motor 204; the shaft may be the same as or similar to the rotating shaft 208; and the rotating insulator may be the same as or similar to the rotating insulator 212. The surgical tool 300 may additionally or alternatively have an extension/retraction mechanism (e.g., for moving the tool bit 304 from the retracted position to the extended position and/or vice versa), and one or more gears, screws, or other devices to enable movement of the tool bit 204 from the retracted position to the extended position and/or vice versa. The surgical tool 300 comprises a tool bit 304, electrical conductors 308, insulators 312, and a housing 316. The electrical conductors 308 may be, for example, RF conductors. In some embodiments, the tool bit 304 may be the same as or substantially similar to the tool bit 216. In other words, the tool bit 304 may be designed to drill, burr, shave, saw, cut, ream, penetrate, and/or tap through anatomical tissue.

The electrical conductors 308 together provide an electrode for the surgical tool 300, which permits the surgical tool 300 to perform electrosurgery. The electrical conductors 308 are positioned on two opposite sides of the tool bit 304 and are pivotally connected to the housing 316. The electrical conductors 308 are biased toward a closed position, with the electrical conductors 308 contacting one another when the tool bit 304 is in the retracted position. The electrical conductors 308 conduct electrical current received via, for example, a wire (not shown) that extends through the housing 316 or through contact with the housing 316 itself. In some embodiments, the electrical conductors 308 together function as a single electrode (e.g., when in contact with each other), and the surgical tool corresponding to the portion 300 is utilized for monopolar electrosurgery. In other embodiments, only one side of the electrical conductors 308 is connected or connectable to a power source, and the other side is provided simply to enable the tool bit 304 to be completely enclosed during use of the surgical tool 300 for electrosurgery.

The insulators 312 separate the tool bit 304 from the housing 316. The separation between the tool bit 304 and the housing 316 prevents the tool bit 304 from unintentionally conducting current when the surgical tool 300 is in the retracted position, as shown in Fig. 3B. The electrical isolation of the tool bit 304 prevents current flow through the tool bit 304 and any components connected thereto, improving safety, and ensuring that the current flows through the electrical conductors 308 to allow for bipolar electrosurgery functionality of the surgical tool 300. The insulators 312 also support the tool bit 304 within the housing 316, both to facilitate rotation of the tool bit 304 around a central axis thereof, and to prevent the tool bit 304 from contacting the housing 316.

In some embodiments, the insulators 312 may be fashioned in a manner that is the same as or similar to that of the rotating insulator 212. In other words, the insulators 312 may uniformly comprise a single insulative material. For instance, in some embodiments, the insulators 312 may be made entirely of a plastic. Alternatively, the insulators 312 may contain a mixture or combination of different types of insulative material. For example, the insulators 312 may comprise a combination of glass and plastic. Additionally or alternatively, the insulators 312 may contain an insulative coating on the surface thereof. The insulative coating may comprise the same or different material than the insulative material. The insulators 312 may comprise, by way of example, insulative material and/or insulative coating made of glass, plastic, porcelain, synthetic resins, paper, Teflon, rubber (natural and/or synthetic), PVC, and/or combinations thereof.

Fig. 3A illustrates the tool bit 304 in the extended position. With the tool bit 304 in the extended position, the surgical tool 300 may operate the same as or similar to that of the surgical tool 200. That is, the surgical tool 300 may be used to perform a mechanical task. In the extended position, a proximal end of the tool bit 304 is located within the housing 316, while a distal end of the tool bit 304 is located outside the housing 316. The distal end of the tool bit 304 may then be placed in contact a surgical site and used to perform a mechanical task or operation, such as drilling through anatomical tissue. In some embodiments, the surgical tool 300 may be incapable of performing electrosurgery in the extended position. As shown in Fig. 3A, the distal end of the tool bit 304 may extend outside the housing 316 and move the electrical conductors 308 into an open position. In the open position, the electrical conductors 308 are separated from one another, which prevents current flow therethrough and removes the ability for the surgical tool 300 to perform electrosurgery. The electrical conductors 308 may comprise a non-conductive panel on a side thereof facing the tool bit 304, so that the tool bit 304 is electrically insulated from the electrical conductors 308.

Fig. 3B shows the tool bit 304 in the retracted position. In the retracted position, both the proximal end and the distal end of the tool bit 304 are located within the housing 316. With the tool bit 304 in the retracted position, the surgical tool 300 is configured for electrosurgery. As the tool bit 304 retracts, the biased electrical conductors 308 return to the closed position. While in the closed position, the electrical conductors 308 contact one another, forming a connection through which current can flow. The closed position of the electrical conductors 308 allows the surgical tool 300 to be used to perform electrosurgery. In some embodiments, the surgical tool 300 may have a switch, button, lever, or the like that permits the operator of the surgical tool 300 to retract the tool bit 304 from the extended position, placing the tool bit 304 into the retracted position, so that the tool bit 304 may be housed within the housing 316. In other embodiments, the tool bit 304 retracts automatically when the operator of the surgical tool 300 switches the tool into an electrosurgery mode (e.g., using a switch, button, lever, or other user interface), and extends automatically when the operator of the surgical tool 300 switches the tool out of the electrosurgery mode.

In some embodiments, during the mechanical operation of the surgical tool 300, the tool bit 304 may be used to selectively cut or remove anatomical tool. When electrosurgery is required or would be beneficial during the surgery, the surgeon or other operator may switch the surgical tool 300 to the electrosurgical function. As a result, the surgical tool 300 may turn off the mechanical function of the tool bit 304 and retract the tool bit 304 into the retracted position.

To perform the monopolar electrosurgery, the operator of the surgical tool 300 may turn on the power source connected to one of or both of the electrical conductors 308 when the tool bit 304 is in the retracted position. Alternatively, the operator may simply select an electrosurgical function, which may cause the surgical tool 300 to retract the tool bit 304 and enable current to flow to the electrical conductors 308. The power source is capable of supplying the necessary current to perform electrosurgery. In some embodiments, the power source may be located external to the surgical tool 300, with the surgical tool 300 operatively connected to the power source. Alternatively, the power source may be located within the housing 316. The power source may be used to selectively power a motor of the surgical tool 300 and the electrical conductors 308 of the surgical tool 300.

Once the power source is activated, the electrical conductors 308 conduct current, allowing for monopolar electrosurgery. The current flows from one electrical conductor 308 and back through to a return electrode (e.g., an electrode located on, around, near, or inside the body of a patient), completing the circuit. Once the monopolar electrosurgical operation is complete, the power source can be turned off (e.g., by a surgeon or operator of the surgical tool 300), and the surgical tool 300 may reconfigure automatically (or be manually reconfigured) for mechanical operation (e.g., to permit use of the tool bit 304 to perform mechanical tasks), including by returning the distal end of the tool bit 304 to outside the housing 316 (e.g., moving the tool bit 304 to the extended position).

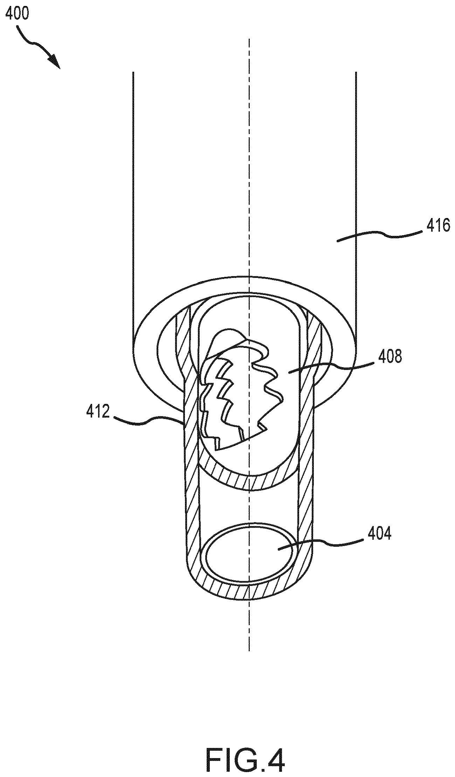

Referring now to Fig. 4, a distal end portion of a surgical tool 400 will be described in accordance with at least one embodiment of the present disclosure. The surgical tool 400 comprises a curette 404, a tool tip 408, insulation 412, and a housing 416. The distal end portion of the surgical tool 400 is configured to allow for bipolar electrosurgery.

The curette 404 may have a sharpened working end and may be useful for scraping or otherwise cleaning an anatomical surface of a patient. The curette 404 may comprise an elongate tube, or may have a distal portion that comprises a tube and a proximal portion that is not a tube. The curette 404 may, in some embodiments, be extendable and retractable.

The tool tip 408 may be the tip of any tool described herein, including a drill bit, a burr, a shaver, a scalpel, a reamer, a saw, or a tap. The tool tip 408 as shown in Fig. 4 is the tip of a shaver with blades that oscillate to excise tissue. Such blades are provided, for example, in the Midas Rex^{®} Spine Shaver Nucleus Removal Set offered by Medtronic. The tool tip 408 may also, in some embodiments, be extendable and retractable. The tool tip 408 may be operatively connected to a motor that may be used to selectively cause the tool tip 408 to rotate or oscillate.

The insulation 412 may provide insulation to the curette 404 and/or the tool tip 408. In some embodiments, the material 412 may prevent inadvertent electrical connection between the curette 404 and the tool tip 408 outside of the aforementioned bipolar electrosurgery.

The housing 416 beneficially assists to hold the components of the tool 400 in place, while shielding those components (but for the working end of the curette 404 and the tool tip 408) from the anatomy, and vice versa, during use of the surgical tool 400. The housing 416 comprises an electrical insulator to prevent electricity from flowing along undesired flow paths (whether within a patient's anatomy or otherwise). The housing 416 may be flexible in some embodiments, or rigid in other embodiments. The housing 416 may have a smooth outer surface to facilitate insertion and withdrawal of the surgical tool 400 into and from the patient's anatomy, respectively, and also to facilitate rotation or other movement of the surgical tool 400 within the patient's anatomy during a surgical procedure (e.g., while a surgeon is maneuvering the surgical tool 400 to achieve any desired modifications to the patient's anatomy with the tool tip 408 and/or the curette 404.

To perform bipolar electrosurgery, the surgical tool 400 is positioned such that the curette 404 and the tool tip 408 contact a surgical site. The surgical tool 400 then enters or is placed into electrosurgery mode. In the electrosurgery mode, the mechanical functionality associated with the surgical tool 400 may be disabled in some embodiments. In other words, any non-electrosurgical features of the surgical tool 400 are disabled. In some embodiments, the surgical tool 400 may contain a button, switch, lever, or the like that facilitates transition between electrosurgery mode and a mechanical mode. In other embodiments, however, the mechanical functionality of the surgical tool 400 may still be available while the electrosurgery features of the surgical tool 400 are activated and/or in use.

Once the surgical tool 400 enters the electrosurgery mode, in some embodiments, it will be appreciated that the current may flow in both directions (e.g., in applications where alternating current may be used). In other embodiments, the current may flow from the curette 404 through the surgical site and into the tool tip 408. In such embodiments, the current may flow in the opposite direction. In other words, the current may flow from the tool tip 408 through the surgical site to the curette 404 (e.g., in applications where direct current may be used).

After the electrosurgical procedure is performed, the surgical tool 400 may exit (or be caused to exit) the electrosurgical mode. The electrical current flow may be stopped, and the surgical tool 400 may transition to a configuration useful for the performance of mechanical tasks. It will be appreciated that in some embodiments, electrical tasks and mechanical tasks may be perform simultaneously. In some embodiments, the button, switch, lever, or the like may be used again to change the configuration of the surgical tool 400 from the electrosurgery mode to the mechanical mode.

Any one or more of the surgical tools 130, 200, 300, and/or 400 may be transitioned from one mode of operation to another mode of operation (or to a joint mode of operation) in any suitable way. For example, in some embodiments, the surgical tool may receive a user input (e.g., via a button, switch, or the like) to change the mode of operation of the surgical tool. Upon receiving the user input, a processor may send a signal to cause the surgical tool to reconfigure for mechanical or electrosurgical operation. In such embodiments, the processor may be located in the surgical tool, or may be alternatively located within a system (e.g., at least one processor 104 of a system 100). In some embodiments, the processor may be two separate processors, with one processor located in the system, and one processor located in the surgical tool. The two separate processors may communicate, based on the user input, to facilitate reconfiguration of the surgical tool.

In some embodiments, a processor may make use of one or more sensors (e.g., one or more sensors of a robot 140, and/or one or more sensors of the surgical tool 130, 200, 300, and/or 400) and, based on data collected from the one or more sensors, send a signal to cause the surgical tool to transition from one mode of operation to a joint mode of operation, such as to reconfigure the surgical tool for mechanical or electromechanical (e.g., a combination of mechanical operation and electrosurgery) operation. In some embodiments, the surgical tool may be configured by a user input (e.g., via user a button, switch, or the like). For instance, the user of the surgical tool may toggle a switch, which may cause the surgical tool to reconfigure from one mode of operation to another mode of operation (or to a joint mode of operation, e.g., an electromechanical operation). In some embodiments, the surgical tool may contain a plurality of buttons, with each button corresponding to a different mode of operation. The user may then press a button to reconfigure the mode of operation of the surgical tool.

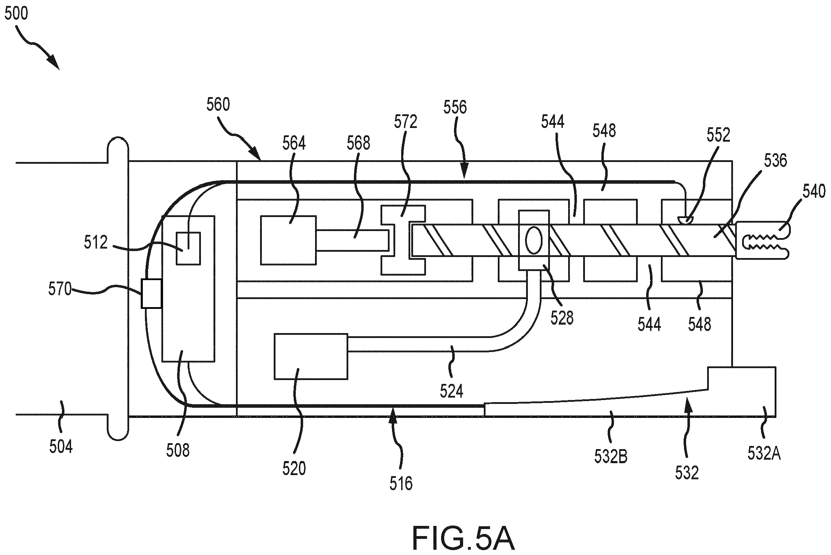

Fig. 5A shows a side view of a surgical tool 500 in accordance with embodiments of the present disclosure. The surgical tool 500 may be the same as or similar to the surgical tool 400, or vice versa. The surgical tool 500 comprises a handle 504 and a housing 560. Provided on or within the housing 560 are a controller 508, a fluid reservoir 520, a curette 532, a spine shaver 536, a processor 570, and an electrical conductor 552. Notwithstanding the layout of the surgical tool 500 as illustrated in Fig. 5A, surgical tools according to other embodiments of the present disclosure may comprise more or fewer components than the surgical tool 500. The processor 570 may be the same as or similar to the processor 104. In some embodiments, for example, the fluid reservoir 520 may include insulative material disposed therearound to electrically isolate the fluid reservoir 520 from the components of the housing 560. Other embodiments of the tool 500 may not include a fluid reservoir 520. For example, in some embodiments, the tool 500 may comprise one or more ports or valves for connecting the tool 500 to an external fluid reservoir.

The housing 560 has a proximal ending operatively connected to and extending from a distal portion of the handle 504 and a distal end that interacts with a patient during a surgical procedure. The handle 504 attaches to the proximal end of the housing 560 and allows a surgeon to more easily grip and/or handle the surgical tool 500. In some embodiments, the handle 504 may contain grooves and/or grips for better handling by the operator. In some embodiments, the handle 504 may be configured to operatively attach to the robot 140, allowing for robot-assisted, automatic, and/or autonomous use of the surgical tool 500.

The housing 560 contains, among other things, the controller 508. The controller 508 provides or enables electrical and mechanical control of the surgical tool 500, as well as signal communication between the surgical tool 500 and, for example, a system 100. For instance, when the surgical tool 500 is in a mechanical mode, the controller 508 may notify the computing device 102 that the driver 564 (described below) is operating and may request instructions for the driver 564 via a communication interface 512 to generate a desired rotation speed or vibration frequency in the spine shaver 536. In this case, the communication interface 512 may contain a processor, which may receive instructions from the computing device 102 associated with the instructions 124 to direct the driver 564 of the surgical tool 500 to operate at a specific frequency or speed (e.g., an ultrasonic oscillation frequency). In other embodiments, the controller 508 may itself control the rotation speed, vibration frequency, or oscillation frequency of the spine shaver 536, without communication with or input from a computing device 102 or another external device.

In some embodiments, the communication interface 512 may be replaced by a switch, button, or other user interface useful for allowing an operator of the surgical tool 500 to select a desired mode of operation. For instance, in some embodiments the communication interface 512 may be replaced by one or more buttons, where each button can be pressed or otherwise selected by the operator of the surgical tool 500 to change the mode of operation of the surgical tool 500. Alternatively, the surgical tool 500 may comprise both a communication interface 512 and a separate user interface via which an operator can selected a desired mode of operation.

In some embodiments, the controller 508 may communicate with a robot 140 via the communication interface 512. For example, if the robot 140 is holding the surgical tool 500, the controller 508 may receive instructions, such as to switch from the mechanical mode to electrosurgery, from the computing device 102. The controller 508 may then carry out the instructions by, for example, powering down the driver 564 and sending electrical current through the surgical tool 500 to permit the performance of electrosurgery.

The housing 560 comprises a driver 564, a shaft 568, and an insulator 572. The driver 564 is attached to a proximal end of the shaft 568, and functions to vibrate or oscillate the shaft 568. In some embodiments, the driver 564 is a magnetic ultrasonic vibration generator. The driver 564 drives the oscillation of the spine shaver 536 by transferring energy through the shaft 568 and the insulator 572 to the spine shaver 536, permitting the surgical tool 500 to be used, for example, to remove nuclear material during a spine surgery. In some embodiments, the driver 564 may be a motor, the shaft 568 may be a vibration shaft (or, e.g., a rotating shaft), and the insulator 572 may be a vibration insulator or a rotating insulator. The shaft 568 may function similarly to or the same as the rotating shaft 208. In other words, the shaft 568 transmits energy from the driver 564 to the spine shaver 536.

The insulator 572 of the surgical tool 500 is located within the housing 560 and is attached to a distal end of the shaft 568. The insulator 572 physically and electrically separates the shaft 568 from the spine shaver 536. The insulator 572 is operatively connected to a proximal end of the spine shaver 536 and transfers the energy received from the driver 564 and the shaft 568 to the spine shaver 536.

The insulator 572 is constructed from an insulative material designed to prevent current from passing therethrough. In some embodiments, the insulator 572 may be the same as or similar to that of rotating insulator 212. In other words, the insulator 572 may uniformly comprise a single insulative material. For instance, in some embodiments, the insulator 572 may be made entirely of a plastic. Alternatively, the insulator 572 may contain a mixture or combination of different types of insulative material. For example, the insulator 572 may comprise a combination of glass and plastic. Additionally or alternatively, the insulator 572 may contain an insulative coating on the surface thereof. The insulative coating may comprise the same or different material than the insulative material. The insulator 572 may comprise, by way of example, insulative material and/or insulative coating made of glass, plastic, porcelain, synthetic resins, paper, Teflon, rubber (natural and/or synthetic), PVC, and/or combinations thereof. The insulator 572 may be made from a material that does not or will not dampen or otherwise absorb vibrations, to ensure that vibrations generated by the driver 564 are passed to the spine shaver 536.

In some embodiments, both the shaft 568 and the insulator 572 may comprise material that can withstand the vibrations or oscillations generated by the driver 564. For instance, the shaft 568 and the insulator 572 may have materials designed to provide increased resilience to vibrational waves generated by the driver 564, and/or materials designed to mitigate, reduce, or prevent resonance effects within the surgical tool 500.

The spine shaver 536 is located partially within the housing 560 and has a proximal end operatively connected to the insulator 572 and a distal end containing a tool tip 540. The tool tip 540 extends out of the housing 560 and, among other things, assists with removal of nucleus material during spine surgeries. The tool tip 540 receives the vibrations or oscillations generated by the driver 564, which enable the tool tip 540 to be used to among other things, remove nucleus material. In some embodiments, the tool tip 540 may contain blades designed to excise tissue.

In some embodiments, the surgical tool 500 may be configured to include mechanical components different from the spine shaver 536, the tool tip 540, and/or the curette 532. For instance, in some embodiments the tool tip of the surgical tool 500 may be the tip of any tool described herein, including a drill bit, a burr, a shaver, a scalpel, a reamer, a saw, or a tap instead of a spine shaver and/or instead of a curette. In such embodiments, the surgical tool 500 may contain additional components (e.g., a motor, a shaft, an insulative support, etc.) that may be used to selectively cause the tool tip to rotate or oscillate.

The spine shaver 536 is surrounded by insulation 548. The insulation 548 serves to electrically insulate the driver 564, the shaft 568, the insulator 572, and the spine shaver 536 from other components in the housing 560. The insulation 548 has extended insulative portions 544 which contact the spine shaver 536. The insulative portions 544 prevent the spine shaver 536 from electrically interacting with the other components in the housing 560 and assist with the alignment of the spine shaver 536 within the housing 560 of the surgical tool 500. In some embodiments, the insulation 548 and the insulative portions 544 may be rigid enough to provide structural support to and fully encompass the driver 564, the shaft 568, the insulator 572, and the spine shaver 536. In some embodiments, the insulation 548 may be the same as or similar to the insulative support 220.

In some embodiments, the insulation 548 and the insulative portions 544 may be made of the same insulative material as or similar insulative material to the rotating insulator 212 and/or insulator 572. That is, the insulation 548 and the insulative portions 544 may uniformly comprise a single insulative material. For instance, in some embodiments, the insulation 548 and the insulative portions 544 may be made entirely of a plastic. Alternatively, the insulation 548 and the insulative portions 544 may be a mixture or combination of different types of insulative material. For example, the insulation 548 and the insulative portions 544 may comprise a combination of glass and plastic. Additionally or alternatively, the insulation 548 and the insulative portions 544 may contain an insulative coating on the surfaces thereof. The insulative coating may comprise the same material as or a different material than the insulative material. The insulation 548 and the insulative portions 544 may comprise, by way of example, insulative material and/or insulative coating made of glass, plastic, porcelain, synthetic resins, paper, Teflon, rubber (natural and/or synthetic), PVC, and/or combinations thereof.

The fluid reservoir 520 holds a fluid (e.g., saline) capable of being transferred to the spine shaver 536 during a surgical procedure. The fluid provided by the fluid reservoir 520 assists in irrigation of the surgical site of the patient during the surgical procedure. For example, during an inter-vertebral disc preparation, a target surgical site may require irrigation to avoid damage to the spine shaver 536 and/or the tool tip 540, or to maintain correct fluid levels in or around the surgical site to prevent an increase in temperature. In some embodiments, the fluid reservoir 520 is a two-way system designed to both provide fluid to a surgical site as well as remove fluid from the surgical site. The fluid reservoir 520 may permit for two-way fluid flow and may have a compartment therein for holding the fluid for application, as well as a compartment for holding the fluid removed from the surgical site. The tool tip 540 may be outfitted to allow for the removal of fluid from the surgical site via suction.

In some embodiments, the surgical tool 500 may be configured to discharge the fluid during the electrosurgical operation of the surgical tool 500. For example, when the surgical tool 500 is configured for electrosurgery (described below), the fluid reservoir 520 may be configured to allow the fluid to flow through the spine shaver 536 and out of a distal end of the tool tip 540. In some embodiments, fluid reservoir 520 may facilitate hemostatic sealing (e.g., Transcollation^{™}). As described herein, hemostatic sealing refers to the discharge of fluid during electrosurgical operation of a surgical tool or device to stop bleeding at a surgical site in anatomical tissue. The operator of the surgical tool 500 may, during performance of electrosurgery at a surgical site, facilitate hemostatic sealing by signaling the fluid reservoir 520 (e.g., via a button, switch, lever, or the like) to pump, transfer, or otherwise release the fluid therefrom. The fluid may be discharged through the tool tip 540, while the tool tip 540 simultaneously operates as an electrode for electrosurgery. The resulting addition of fluid to the surgical site receiving electrosurgery creates hemostasis at the surgical site, without the creation of smoke or char.

The fluid reservoir 520 comprises a pipe 524 and a transfer portion 528. The pipe 524 is in fluidic communication with the transfer portion 528, with the transfer portion 528 transferring the fluid flowing through the pipe 524 from the fluid reservoir 520 to the spine shaver 536. The spine shaver 536 contains a hollow portion through which the fluid flows. The fluid flows toward the distal end of the surgical tool 500 and exits through the tool tip 540. In some embodiments, the hollow portion of the spine shaver 536 may be forward biased, such that fluid flows in a distal direction toward the tool tip 540.

In some embodiments, the fluid reservoir 520 may contain a pump. The pump may be configured to move the fluid contained in the fluid reservoir 520 through the pipe 524 and into the transfer portion 528. In some embodiments, the fluid reservoir 520 may include a port for refilling the fluid reservoir. The port may be configured to receive fluid from a fluid source external to the surgical tool 500. As such, the fluid reservoir may be refillable, such that the surgical tool 500 maintains an adequate amount of fluid. In some embodiments, the fluid reservoir 520 may be external to the surgical tool 500, and the pipe 524 may extend out of the surgical tool 500 and connect the fluid reservoir 520 to the surgical tool 500.

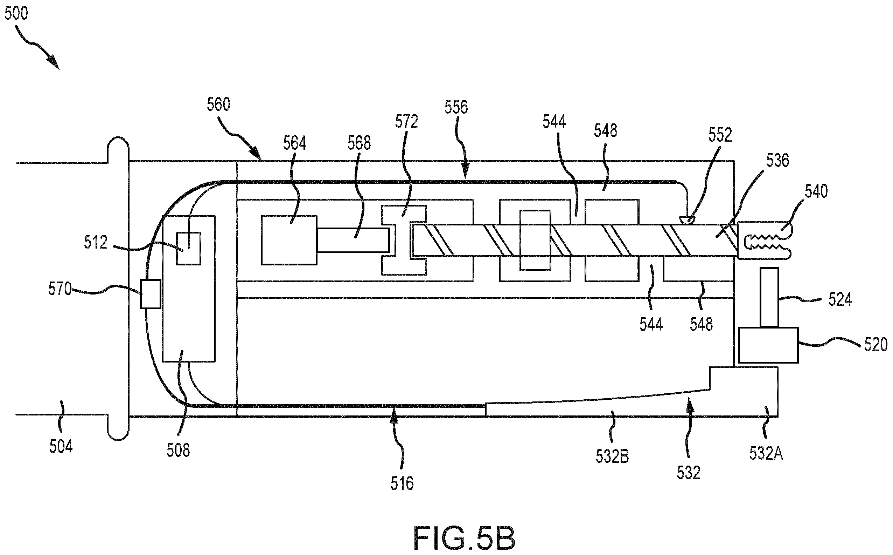

In some embodiments, as shown in Fig. 5B, the fluid reservoir 520 and the pipe 524 may be outside of the housing 504. In such embodiments, fluid may be delivered from the fluid reservoir 520 to the tool tip 540 via the pipe 524.

In some embodiments, the transfer portion 528 may contain a waterproofing ring and/or waterproofing material to prevent the fluid from entering, contacting, or otherwise fluidically communicating with other components in the housing 560. The waterproofing ring may be circumferentially disposed about the spine shaver 536, which may prevent the fluid from leaking out of the spine shaver 536. In some embodiments, the transfer portion 528 may contain a valve. The valve may selectively release the fluid into the spine shaver 536. In some embodiments, the valve controlled by the controller 508 such that the controller 508 modulates the parameters (e.g., time of opening, frequency of opening, amount of fluid released) related to the dispersion of fluid from the fluid reservoir 520 to allow for a variety of controlled flow rates and fluid dispersion.

The curette 532 of the surgical tool 500 is partially disposed within the housing 560. The curette 532 comprises a distal end 532A which extends out of the distal end of the housing 560, and a proximal end 532B which extends toward the proximal side of the housing 560. The curette 532 connects to a wire 516, the wire 516 in electric communication with the controller 508. The wire 516 conducts current from the controller 508 to the curette 532. In some embodiments, the wire 516 may be insulated such that any current in the wire 516 flows between the controller 508 and the curette 532 and is prevented from electrically contacting other components in the housing 560. In some embodiments, the curette 532 may be similar to or the same as the curette 404. In such embodiments, the curette 532 may have a sharpened working end and be useful for scraping or otherwise cleaning an anatomical surface of a patient.

The electrical conductor 552 of the surgical tool 500 provides an electrode for conducting electrical current between the controller 508 and the spine shaver 536. While the curette 532 is not intended to move and therefore may be attached directly to the wire 516, the spine shaver 536 vibrates, oscillates, and/or rotates. Use of the electrical conductor 552 ensures electrical contact with the spine shaver 536 despite such movement. The wire 556 runs through the insulation 548 to connect the controller 508 to the electrical conductor 552. The electrical conductor 552 and the curette 532 permit the surgical tool 500 to perform bipolar electrosurgery. In some embodiments, the electrical conductor 552 may be the same as or similar to the RF lead 228. In some embodiments, the electrical conductor 552 is retractable such that when the surgical tool 500 performs a mechanical operation using the spine shaver 536 and/or the curette 532, the electrical conductor 552 may be in a retracted position and not electrically contacting the spine shaver 536. When the surgical tool 500 performs bipolar electrosurgery, the electrical conductor 552 may move to an extended position and contact the spine shaver 536 to permit current flow.

To perform the bipolar electrosurgery, the surgical tool 500 is positioned such that the curette 532 and the tool tip 540 contact a target surgical site. A surgeon or other operator may then activate an electrosurgical mode of the surgical tool 500. In some embodiments, the surgical tool 500 may comprise a button, switch, lever, or the like that may be used by the operator of the surgical tool 500 to signal the change into the electrosurgery mode. In some embodiments, in the electrosurgery mode, the mechanical functionality of the surgical tool 500 (i.e., the operation of the spine shaver 536 via the driver 564) may be disabled. In such embodiments, the controller 508 may confirm that the mechanical functionality of the surgical tool 500 is disabled by turning off the driver 564. In some embodiments, the controller 508 may send a signal through the communication interface 512 to the computing device 102 to indicate that the surgical tool 500 is in electrosurgery mode.

Once the surgical tool 500 has entered or been configured to be in the electrosurgery mode, electrical signals are sent from the processor 570 through the wires 516, 556 to the curette 532 and the electrical conductor 552, respectively. The current then flows through the target surgical site. In some embodiments, the current may flow in either direction; for instance, the current may flow from the curette 532 to the tool tip 540 and through the electrical conductor 552 or from the electrical conductor 552 through the tool tip 540 to the curette 532 (e.g., in embodiments where direct current may be used). In other embodiments, it will be appreciated that the current may flow in both directions (e.g., in embodiments where alternating current may be used).

After performing the bipolar electrosurgery, the operator of the surgical tool 500 may cause the surgical tool 500 to exit the electrosurgery mode. The controller 508 disables the flow of current through the wires 516, 556, which halts the flow of current through the target surgical site. In some embodiments, the exit from the electrosurgery mode may reactivate the use of the driver 564. In some embodiments, the button, switch, lever, or the like may be used by the operator of the surgical tool 500 to signal the change out of the electrosurgery mode.

In some embodiments, the surgical tool 500 may allow for the use of the curette 532 outside of the mechanical and/or electrosurgery mode for, among other things, the removal of anatomical tissue through scraping. In such embodiments, the surgical tool 500 may operate as a non-powered tool. In some embodiments, an electrosurgery device (e.g., the bipolar electrosurgery of the surgical tool 500) may be used by a surgeon or operator of the surgical tool 500 on a surgical site before any mechanical operations (e.g., drilling using a tool bit) are performed. By applying electrosurgery to anatomical tissue before implementing a mechanical operation of the surgical tool 500, the anatomical tissue may be loosened, softened, or otherwise altered by the electrosurgery prior to the introduction of mechanical tools such that the anatomical tissue is easier to remove with mechanical operations of the surgical tool 500.

In some embodiments, the controller 508 may communicate with the computing device 102 to provide an operator with the user interface 112. The user interface 112 may provide the operator with different modes for the surgical tool 500 as well as the option to switch therebetween. For example, the user interface 112 may provide the user with an off mode, a shaver mode, an electrosurgery mode, and an electromechanical mode. While the surgical tool 500 is in the off mode, no power may flow to the surgical tool 500, but the surgical tool 500 may still operate as a scraper, with the curette 532 capable of scraping, cutting, cleaning, or otherwise modifying anatomical tissue. When the shaver mode is selected (e.g., through user input via a button, switch, or the like, or via a signal from a processor based on data gathered from one or more sensors), the surgical tool 500 may be able to perform a mechanical operation. In the shaver mode, the driver 564 may power the spine shaver 536, permitting the tool tip 540 to, for example, remove and/or modify anatomical tissue. When the electrosurgery mode is selected, the mechanical operation of the surgical tool 500 may be disabled. For example, the surgical tool 500 may disable the driver 564 (e.g., via a signal sent by a processor), which may stop and further prevent operation of the spine shaver 536 and/or the tool tip 540 while the surgical tool 500 is in the electrosurgery mode. When the operator of the surgical tool 500 selects the electrosurgery mode, the surgical tool 500 may reconfigure or be reconfigured to permit the operator of the surgical tool 500 to perform bipolar electrosurgery. When the electromechanical mode is selected, the surgical tool 500 may reconfigure to perform both electrosurgery and mechanical operation. In other words, in the electromechanical mode, in addition to the driver 564 causing motion or vibration in the spine shaver 536 , the tool tip 540 and/or the curette 532 may also receive electrical current such that bipolar electrosurgery may be performed upon the electrical connection of the tool tip 540 and the curette 532 through the target surgical site. The simultaneous operation of the driver 564 and the current flow to both the spine shaver 536 and the curette 532 may permit the operator of the surgical tool 500 to simultaneously perform the mechanical operation and electrosurgery.

Further examples of the invention may be as follows:
Example 1 is a dual-mode surgical system, comprising: a housing having a proximal end and a distal end; a mechanical tool bit supported at least partially within the housing; an electrosurgery device supported by the housing, the electrosurgery device comprising at least one electrical conductor; and a fluid conduit for channeling fluid to proximate the distal end.
In Example 2, the dual-mode surgical system of Example 1 can optionally further include a valve for selectively releasing fluid from the fluid conduit.
In Example 3, the dual-mode surgical system of Example 1 or 2 can optionally further include that the mechanical tool bit comprises an electrode of the electrosurgery device.
In Example 4, the dual-mode surgical system of any one of Examples 1 to 3 can optionally further include that the mechanical tool bit is movable between a retracted position and an extended position.
In Example 5, the dual-mode surgical system of any one of Examples 1 to 4 can optionally further include that the at least one electrical conductor comprises first and second elements hingedly connected to the housing, each of the first and second elements moveable between an open position, in which the first element does not contact the second element, and a closed position, in which the first element is in contact with the second element.
In Example 6, the dual-mode surgical system of Example 5 can optionally further include that the first and second elements are biased toward the closed position.
Example 7 is a surgical tool comprising: a housing; a first tool bit operably connected to a motor and supported by the housing, the first tool bit in electrical contact with a first electrical lead; a second tool bit supported by the housing and insulated from the first tool bit within the housing, the second tool bit in electrical contact with a second electrical lead different than the first electrical lead; and a user interface operable to select a non-powered mode in which the second tool bit may be used to modify anatomical tissue, a first powered mode in which the first tool bit may be used to modify anatomical tissue, or a second powered mode in which the first tool bit and the second tool bit are used to apply electrical current to anatomical tissue.
In Example 8, the surgical tool of Example 7 can optionally further include that one of the first tool bit and the second tool bit comprises a fluid conduit for discharging fluid proximate a working end thereof.
In Example 9, the surgical tool of Example 7 or 8 can optionally further include that the first tool bit is a spine shaver and the second tool bit is a curette.
In Example 10, the surgical tool of any one of Example 7 to 9 can optionally further include that the spine shaver comprises a fluid conduit operable to discharge fluid in the second powered mode.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the description has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

## Claims

1. A surgical tool comprising:
a mechanical tool for selectively cutting or removing anatomical tissue, comprising:
a tool bit; and
a driver operatively connected to the tool bit; and
an electrosurgery device selectively operable to apply an electric current to anatomical tissue, comprising:
a first electrode; and
a second electrode different than the first electrode.

2. The surgical tool of claim 1, wherein at least one of the first electrode and the second electrode is retractable from a first position used when the electrosurgery device is operated to a second position used when the mechanical tool is operated.

3. The surgical tool of claim 1 or 2, wherein the first electrode is the tool bit.

4. The surgical tool of any one of claims 1 to 3, wherein the tool bit is insulated from the first electrode and the second electrode.

5. The surgical tool of any one of claims 1 to 4, wherein activation of the driver deactivates the electrosurgery device.

6. The surgical tool of any one of claims 1 to 5, wherein the driver and the electrosurgery device are operable simultaneously.

7. The surgical tool of any one of claims 1 to 6, wherein the tool bit comprises a drill bit, a burr, a saw, an ultrasonic scalpel, or a shaver.

8. The surgical tool of any one of claims 1 to 7, further comprising a fluid conduit for discharging fluid proximate a distal end of the electrosurgery device.

9. The surgical tool of any one of claims 1 to 8, wherein extension of the tool bit causes at least one of the first electrode and the second electrode to move from an operating position to a non-operating position.

10. The surgical tool of claim 9, wherein the at least one of the first electrode and the second electrode is biased toward the operating position.
